(19) Europäisches Patentamt
European Patent Office
Office européen des brevets

(11) Veröffentlichungsnummer: **0 336 248**
**A1**

# (12) EUROPÄISCHE PATENTANMELDUNG

(21) Anmeldenummer: **89105353.0**

(22) Anmeldetag: **25.03.89**

(51) Int. Cl.4: **C07D 493/10 , A61K 31/35 , //(C07D493/10,311:00,307:00)**

(30) Priorität: **31.03.88 DE 3811016**
**07.09.88 DE 3830324**

(43) Veröffentlichungstag der Anmeldung:
**11.10.89 Patentblatt 89/41**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim (Taunus)(DE)**
Erfinder: **Grabley, Susanne, Dr.**
**Hölderlinstrasse 7**
**D-6240 Königstein/Taunus(DE)**
Erfinder: **Seibert, Gerhard, Prof. Dr.**
**Gläserweg 21**
**D-6100 Darmstadt(DE)**
Erfinder: **Winkler, Irvin, Dr.**
**In den Eichen 40**
**D-6237 Liederbach(DE)**

(54) **Am F-Ring substituierte Nigericinderivate, Verfahren zu deren Herstellung, diese enthaltende Mittel und Verwendung derselben als antiviral wirksam und antibakteriell wirksame Substanzen.**

(57) Die Erfindung betrifft Nigericinderivate der Formel I

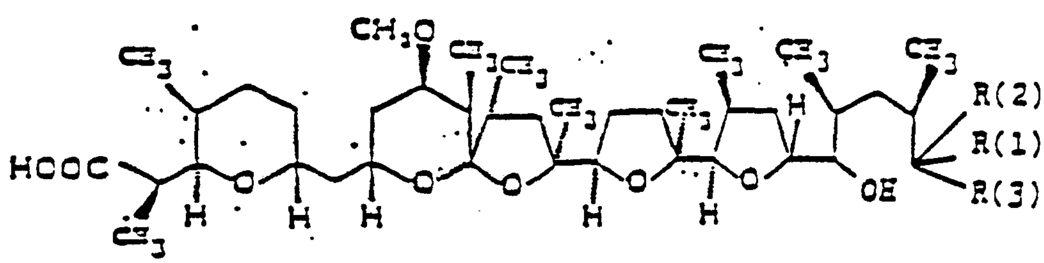

in denen R(1), R(2) und R(3) die angegebenen Bedeutungen haben. Diese Verbindungen wirken antibakteriell und antiviral.



Xerox Copy Centre

**Am F-Ring substituierte Nigericinderivate, Verfahren zu deren Herstellung, diese enthaltende Mittel und Verwendung derselben als antiviral wirksam und antibakteriell wirksame Substanzen.**

Der Polyether Nigericin, der durch Kultivierung von Streptomyces hygroscopicus erhalten werden kann, ist u. a. beschrieben von J. Berger et al., Am. Chem. Soc. 73, 5295 (1951)

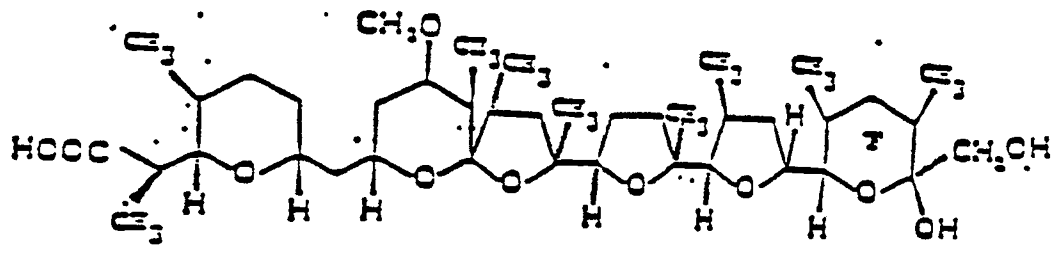

und wurde bislang als Antibiotikum eingesetzt.

Einige Derivate des Nigericins sind ebenfalls bekannt (JP 72 01 288, US 38 32 358 und Tokuo Kubota et al., J. Chem. Soc. (C), 1970, 695) auch sie wurden bislang als Antibiotika eingesetzt.

Eine antivirale Wirksamkeit von Nigericin oder Nigericinderivaten ist bisher noch nicht bekannt geworden. Lediglich in der älteren deutschen Anmeldung P 3800598 ist eine solche antivirale Wirksamkeit vorgeschlagen worden; diese ist jedoch in vielerlei Hinsicht noch nicht befriedigend.

Es wurde nun gefunden, daß bestimmte Nigericinderivate hochwirksame antivirale und antibakterielle Mittel sind.

Die Erfindung betrifft Nigericinderivate der Formel I

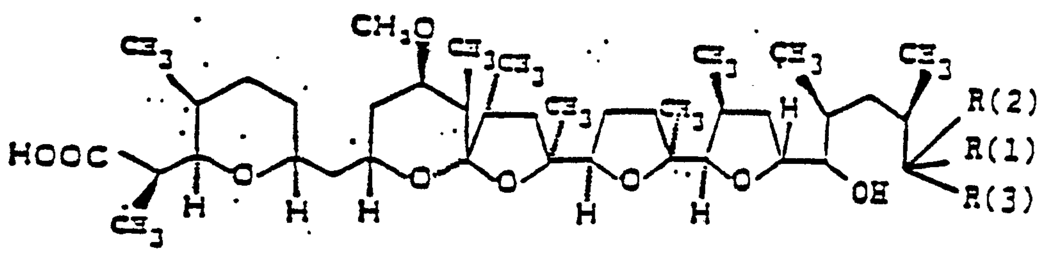
I

in denen

R(1) Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert durch ($C_1$-$C_{10}$)-Alkyl, ($C_1$-$C_{10}$)-Alkoxy oder Phenoxy,

R(2) Wasserstoff, ($C_1$-$C_{18}$)-Alkyl (geradkettig oder verzweigt, gesättigt oder ungesättigt), ($C_3$-$C_8$)-Cycloalkyl, Phenyl, $CH_2OH$, wobei das Phenyl unsubstituiert ist oder substituiert durch ($C_1$-$C_{10}$)-Alkyl, ($C_1$-$C_{10}$)-Alkoxy oder Phenoxy,

R(3) OH,

bedeuten,

oder in der

R(2) und R(3) gemeinsam Sauerstoff und

R(1) Wasserstoff, ($C_1$-$C_{18}$)-Alkyl (geradkettig oder verzweigt, gesättigt oder ungesättigt), ($C_3$-$C_8$)Cycloalkyl, Phenyl (unsubstituiert oder substituiert durch ($C_1$-$C_{10}$)-Alkyl, ($C_1$-$C_{10}$)-Alkoxy oder Phenoxy), NR(4)R(5) bedeuten, worin

R(4) und R(5) gleich oder verschieden sind und folgende Bedeutung haben:

a) Wasserstoff,

b) ($C_1$-$C_{18}$) Alkyl (gesättigt oder ungesättigt, geradkettig oder verzweigt), wobei die Alkylgruppen unsubstituiert sind oder substituiert mit F, Cl, Br, Phenyl, Naphthyl, Thienyl, oder CON(R(6))2, oder COOR-(6) mit gleich Wasserstoff, ($C_1$-$C_6$)-Alkyl

c) ($C_3$-$C_8$)-Cycloalkyl,

d) Arylgruppen mit insgesamt 6-20 C-Atomen, worin Aryl Phenyl, Naphthyl, Thienyl bedeutet, welche Systeme entweder unsubstituiert sind oder substituiert mit geradkettigem oder verzweigtem Alkyl mit bis zu 14 C-Atomen, oder F,Cl,Br,I, Nitro, Cyano, Alkoxy, Phenoxy,

sowie die physiologisch verträglichen Salze dieser Verbindungen, mit Ausnahme der Verbindung, in welcher gleichzeitig R(1) Wasserstoff und R(2) $CH_2OH$ und R(3) OH bedeuten; und mit Ausnahme der Verbindungen, in denen gleichzeitig R(2) und R(3) gemeinsam Sauerstoff und R(1) Wasserstoff oder Methyl bedeuten.

Die Erfindung betrifft bevorzugt Verbindungen, in denen mindestens einer der Substituenten folgende Bedeutung hat:

R(1) Wasserstoff, ($C_1$-$C_4$)-Alkyl, Phenyl
R(2) $CH_2OH$
R(3) OH.

Besonders bevorzugt sind Verbindungen 1, in denen R(1) und R(2) gleich sind und Wasserstoff, ($C_1$-$C_6$)-Alkyl oder Phenyl,
und
R(3) OH
bedeuten.

Ebenfalls bevorzugt sind solche Verbindungen, in denen R(2) und R(3) gemeinsam Sauerstoff und
R(1) NR(4)R(5)
bedeuten,
wobei R(4) und R(5) ($C_1$-$C_6$) Alkyl, Phenyl oder Wasserstoff bedeuten.

Insbesondere sind Verbindungen bevorzugt, bei denen R(2) und R(3) gemeinsam Sauerstoff und R(1) Wasserstoff, ($C_1$-$C_7$)-Alkyl oder Phenyl bedeuten.

Die Verbindung I mit R(1) gleich Wasserstoff R(2) gleich $CH_2OH$ und R(3) gleich OH (Verbindung Nr. 2 der Beispiele) ist als Wachstumsförderer bei Rindern bekannt, ebenso ist die Verbindung mit R(2) und R(3) gemeinsam gleich Sauerstoff und R(1) gleich Wasserstoff bekannt (Verbindung 10 der Beispiele); ebenso ist für die obengenannten Verbindungen wie auch für die Verbindung mit R(2) und R(3) gemeinsam gleich Sauerstoff und R(1) = Methyl (Verbindung 11 der Beispiele) die antibakterielle Wirkung schon bekannt.

Gemäß der vorliegenden Erfindung wurde nun gefunden, daß diese Verbindungen auch hervorragend antivirale Wirksamkeiten aufweist.

Sämtliche bis zu 3 im Molekül I vorhandenen OH-Gruppen können frei oder geschützt sein, daß heißt der Wasserstoff der OH-Gruppe kann ersetzt sein durch die Gruppen -CO-R(7), -CO-NH-R(7) oder -CO-O-R(7),
in denen
R(7) ($C_1$-$C_{15}$)-Alkyl bedeutet, welches unsubstituiert oder F-, Cl-, Br-, I-, nitro, cyano, ($C_1$-$C_4$)-alkoxy-, phenyloxy- oder phenylsubstituiert ist, wobei die genannten Phenylreste ihrerseits unsubstituiert oder mit F, Cl, Br, I, Nitro, Cyano, ($C_1$-$C_4$)-Alkyl oder ($C_1$-$C_4$)-Alkoxy substituiert sind, oder in denen
R(7) Phenyl, Furyl oder Thienyl bedeutet, wobei diese Phenyl-, Furyl- oder Thienyl-Reste unsubstituiert oder mit F, Cl, Br, I, Nitro, Cyano, ($C_1$-$C_4$)-Alkyl oder ($C_1$-$C_4$)-Alkoxy substituiert sind.

Die erfindungsgemäßen Verbindungen I werden nach einem Verfahren erhalten, bei welchem man Nigericin

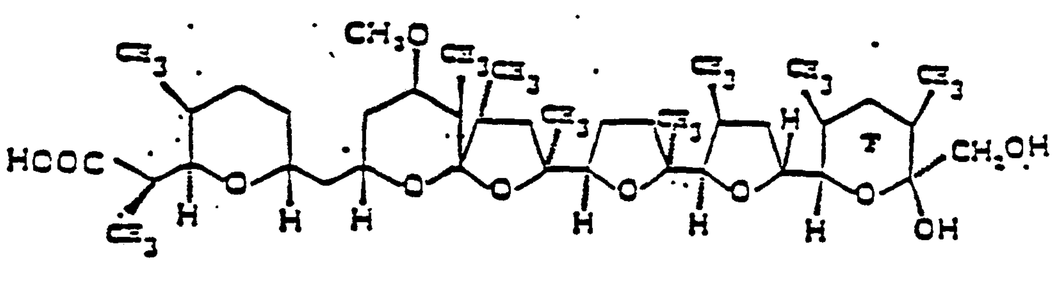

mit einer Verbindung II
R(1)MgHal
umsetzt, in welcher R(1) Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert durch ($C_1$-$C_{10}$)-Alkyl, ($C_1$-$C_{10}$)-Alkoxy oder Phenoxy, und Hal Chlor, Brom oder Iod bedeuten. Ein weiteres Verfahren zum Herstellen der erfindungsgemäßen Verbindungen I besteht darin, daß man Nigericin mit Natriumperiodat zu Verbindungen III

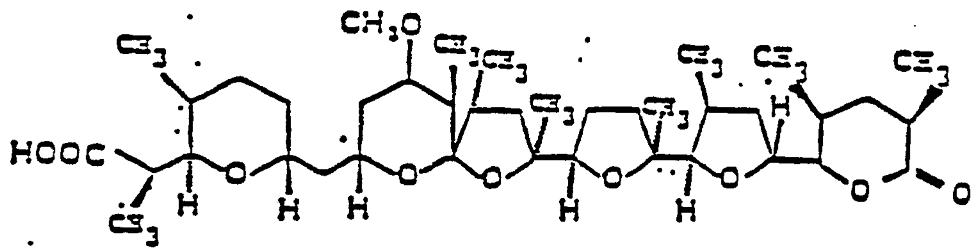

(III)

spaltet und diese Verbindungen III analog zu der vorher beschriebenen Verfahrensweise mit R(1)MgHal zu Verbindungen I umsetzt.

Die erfindungsgemäße Verbindung I mit R(1) = Wasserstoff, R(2) = Wasserstoff und R(3) = OH kann aus der erwähnten Verbindung III durch Reduktion mit Hydridüberträgern erhalten werden.

Erfindungsgemäße Verbindungen I, in denen R(2) und R(3) gemeinsam Sauerstoff bedeuten, lassen sich nach einem Verfahren herstellen; bei welchem Verbindung III, wie beschrieben, hergestellt werden, und bei welchem anschließend diese Verbindungen III mit Aminen HNR(4)R(5) mit R(4) und R(5), wie oben definiert, umgesetzt werden.

Verbindungen I mit R(1) = Wasserstoff, ($C_1$-$C_{18}$)-Alkyl (geradkettig oder verzweigt, gesättigt oder ungesättigt), ($C_3$-$C_8$)Cycloalkyl, Phenyl (unsubstituiert oder substituiert durch ($C_1$-$C_{10}$)-Alkyl, ($C_1$-$C_{10}$)-Alkoxy oder Phenoxy),
und mit R(2) und R(3) gemeinsam gleich Sauerstoff werden erhalten durch Natriumperiodat-Spaltung von Verbindungen I mit R(1) wie soeben definiert und R(2) = $CH_2OH$ und R(3) = OH.

Diese Verbindungen I mit R(2) und R(3) gemeinsam gleich Sauerstoff liegen im Gleichgewicht mit ihrer cyclischen Hemiacetalform Ia vor:

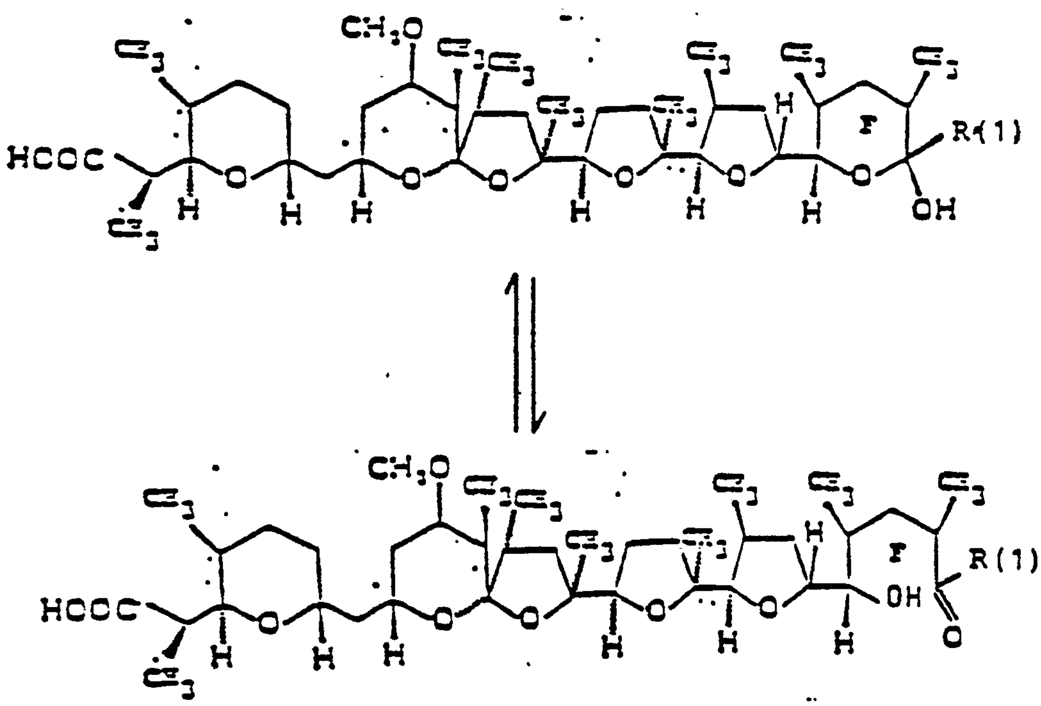

Ia

I

Die Erfindung betrifft weiterhin ein Zwischenprodukt III

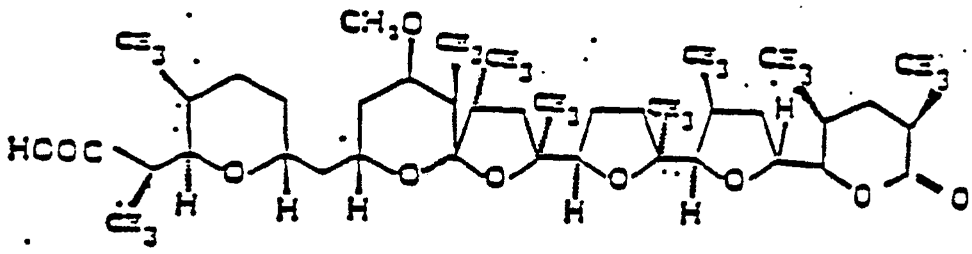 III,

welches sich hervorragend zur Herstellung der erfindungsgemäßen Verbindungen I eignet.

Die Verbindungen I sowie die physiologisch verträglichen Salze dieser Verbindungen haben sich als überragende antivirale und antibakterielle Mittel herausgestellt.

Auch Verbindung III sowie deren physiologisch verträgliche Salze zeigen antibakterielle und antivirale Wirksamkeit.

Im folgenden werden die Verfahren a) bis d) näher beschrieben, nach welchen die erfindungsgemäßen Verbindungen I erhalten werden.

Bei der Verfahrensvariante a) geht man am besten so vor, daß man Nigericin oder das Lacton III mit einem Überschuß einer Grignard-Verbindung der Formel II in einem inerten Lösungsmittel, bevorzugt in einem Ether, wie Tetrahydrofuran, Dioxan oder Diethylether, bis zur Beendigung der Reaktion umsetzt, gegebenenfalls in Gegenwart von Magnesiumhalogeniden, vorzugsweise Magnesiumchlorid.

Die Reaktionstemperaturen liegen dabei ziwschen -70° C und +70° C, insbesondere ziwschen -70° C und +40° C.

Die Reaktionszeiten betragen 1-180 Stunden, bevorzugt 10-48 Stunden. Die Beendigung der Reaktionen kann beispielsweise mit Hilfe der Dünnschichtchromatographie festgestellt werden.

Die Ausgangsverbindungen für die Verfahrensvariante a), die Verbindungen der Formel II, sind, sofern sie nicht käuflich sind, auf einfache Weise nach literaturbekannten Verfahren herstellbar. Beispielsweise erhält man die Grignard-Verbindungen durch Umsetzung der entsprechenden Alkyl- oder Arylhalogenide mit Magnesium in Ether.

Bei der Verfahrensvariante b) geht man am besten so vor, daß man Nigericin mit einem Überschuß an Natriumperiodat nach bekannten Verfahrensweisen umsetzt. Als Lösungsmittel dienen die für die Spaltung mit Natriumperiodat üblichen Lösungsmittel, die literaturbekannt sind; z.B. eigenen sich Alkohole oder fast alle anderen mit Wasser mischbaren Lösungsmittel. Bevorzugt ist ein Gemisch aus Tetrahydrofuran und Wasser.

Die weitere Umsetzung mit der Grignard-Verbindung nach Variante b) erfolg wie oben für Variante a) beschrieben.

Nach Verfahrensvariante c) setzt man vorteilhaft die Verbindung III mit einem Überschuß an Hydridüberträgern um.

Neben Lithiumaluminiumhydrid sind Natriumborhydrid und Natriumcyanoborhydrid bei erhöhten Temperaturen sehr geeignet.

Geeignete Lösungsmittel sind besonders Ether, wie Tetrahydrofuran, Dioxan oder Diethylether. Die Reaktionsbedingungen für die Reduktion mit dem Hydridüberträger werden so gewählt, daß die Reaktion ausschließlich bzw. ganz überwiegend am sogenannten F-Ring des Nigericins stattfindet, und daß die Carboxylgruppe auf der anderen Seite des Moleküls unverändert bleibt. Als geeignet für diese Führung der Reaktion haben sich Temperaturen von -30 bis +40° C erwiesen, insbesondere solche von 0 bis 20° C. Die Reaktionszeiten liegen je nach Reaktionstemperaturen zwischen 1 und 60 Stunden, bevorzugt zwischen 4 und 12 Stunden. Ganz besonders geeignet ist Lithiumalanat als Reduktionsmittel. Im übrigen sind Reduktionen mit Hydridüberträgern literaturbekannte Reaktionen.

Nach Variante d) stellt man, wie oben beschrieben, zunächst die Verbindung III her setzt diese dann mit einem Amin HNR(4)R(5) um. Vorteilhaft ist hierbei ein bis zu 50-facher Überschuß des Amins als Lösungsmittel, oder man setzt ein inertes Lösungsmittel ein, z.B. Chloroform, Methylenchlorid, Tetrahydrofuran oder Dioxan. Vorteilhaft ist es auch, die Reaktion in Gegenwart einer Base durchzuführen. Als besonders vorteilhaft hat es sich erwiesen, die Gesamtreaktion in Tetrahydrofuran auszuführen; als zuzusetzende Base ist Dimethylaminopyridin besonders geeignet. Die Reaktionstemperaturen liegen zwischen -70° C und +70° C, bevorzugt zwischen 0 und 30° C. Die Raktionszeiten liegen zwischen 5 und 20 Stunden.

Bei der Verfahrensvariante e) geht man so vor, daß man die Verbindunngen, die nach Verfahrensvariante a) durch Addition von Grignard-Verbindungen an Nigericin entstehen, nach Verfahrensvariante b) mit Natriumperiodat spaltet.

Verbindung III

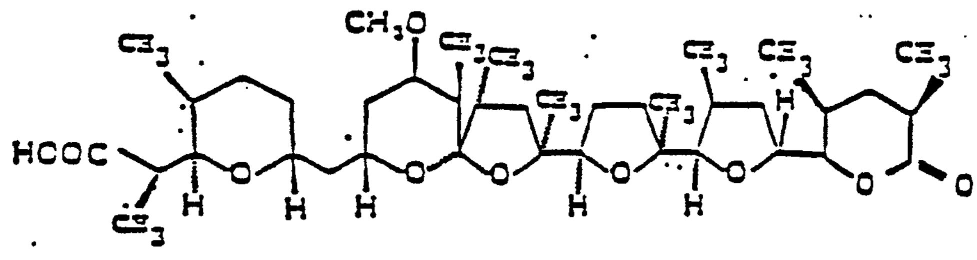

III

wird hergestellt wie in Verfahrensvariante b) beschrieben. Verbindung III weist selbst eine antivirale und antibakterielle Wirkung auf; sie ist hervorragend als Zwischenprodukt zur Herstellung der erfindungsgemäßen Verbindungen I geeignet.

Die Herstellung der physiologisch verträglichen Salze erfolgt nach an sich bekannten Methoden durch Umsetzung mit anorganischen oder organischen Säuren oder Basen. Zur Salzbildung sind insbesondere geeignet Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure Malonsäure, Fumarsäure, Oxalsäure, Zitronensäure, Äpfelsäure, Schleimsäure, Benzoesäure, Salicylsäure, Acetursäure, Naphthalin-1,5-disulfonsäure, Ascorbinsäure, Phenylessigsäure, ß-Aminosalicylsäure, Hydroxyethinsulfonsäure, Benzolsulfonsäure oder synthetische Harze, die saure Gruppen enthalten, z. B. solche mit Ionenaustauscherwirkung und Alkali- und Erdalkalihydroxyde, -carbonate oder -bicarbonate sowie physiologisch verträgliche organische Verbindungen, die eine primäre, sekundäre oder tertiäre Aminogruppe tragen.

Die Reinigung, Isolierung und Aufarbeitung der Substanzen erfolgt nach den üblichen Methoden, beispielsweise können die Reaktionsprodukte durch Chromatographie an polaren Trägermaterialen wie Kieselgel oder ®Sephadex LH 20 mit Lösungsmitteln wie niederen Alkanolen wie Methanol oder Chloroform oder Essigester oder Methanol/Chloroform-Mischungen, aber auch extraktive Methoden wie flüssig/flüssig-Extraktion oder fest/flüssig-Extraktion oder durch Kristallisation gereinigt werden.

Die antivirale Aktivität der erfindungsgemäßen Verbindungen I und III sowie deren physiologisch verträglicher Salze wurde in Zellkulturen, die mit Testviren infiziert wurden, getestet. Dabei zeigte sich, daß die erfindungsgemäßen Derivate eine hervorragende antibakterielle Wirkung zeigen.

Die erfindungsgemäßen Verbindungen sind aufgrund ihrer pharmakologischen Eigenschaften für die Behandlung von bakteriellen Erkrankungen und Viruserkrankungen, die hervorgerufen werden beispielsweise durch HSV I, II (Herpes simplex I- oder II-Virus) oder auch Picorna- und Retroviren, wie HIV (Human Immunodeficiency Virus), geeignet.

Die Erfindung betrifft daher weiter die Anwendung der erfindungsgemäßen Verbindungen der Formel I sowie deren physiologisch verträglicher Salze bei der Behandlung und Prophylaxe von bakteriellen Erkrankungen oder von Herpes-Virus-, Picorna- und Retroviren-Erkrankungen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tag, vorzugsweise 0,001 -1,0 mg/kg/Tag, inbesondere 0,005 - 0,2 mg/kg/Tag, appliziert werden. Besonders bevorzugt ist die topische Anwendung, wobei die Wirkstoffkonzentration in den Salben 0,001 - 1 %, bevorzugt 0,01 - 0,1 %, beträgt. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt. Bei der topischen Anwendung reichen schon Wirkstoffkonzentrationen von 0,001 - 1 %, bevorzugt 0,01 -0,1 %,

aus.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral (subkutan), topisch oder rectal appliziert werden, wobei die topische Applikation bevorzugt ist.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen, Cremes oder Salben. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

In den nachfolgenden Tabellen sind die gemäß den vorstehend beschriebenen Verfahren a bis e synthetisierten Verbindungen aufgeführt. In Tabelle 1 sind die verschiedenen Substituenten R(1), R(2), R(3), R(4) und R(5) der Nigericinderivate zwecks Identifizierung der Verbindungen angegeben. Ebenfalls angegeben in Tabelle 1 ist das Molekulargewicht sowie die Ausgangsverbindung, die Reaktionszeit, das Herstellungsverfahren und die chemische Ausbeute. In Tabelle 2 sind ausgewählte, charakteristische analytische Daten der erhaltenen Verbindungen angegeben (C-, H-Analysen, Molmassenpeak der Natriumverbindung im Massenspektrum und $^{13}C$ NMR-Daten).

Tabelle 3 zeigt die antivirale Wirkung der Verbindungen I,

Tabelle 4 die antibakterialle Wirkung.

Verfahren a

1 mmol Nigericin oder Nigericinderivat wird in 50 ml Tetrahydrofuran gelöst und bei 0 °C 30 h mit 8 mmol Grignardreagenz gerührt. Nach der Hydrolyse mit 50 ml Wasser wird das Tetrahydrofuran im Vakuum abdestilliert. Die wäßrige Phase wird mit 3 x 50 ml 20 Ethylacetat extrahiert, die organische Phase wird mit 0,1 N Salzsäure gewaschen und anschließend über Natriumsulfat getrocknet. Nach dem Abziehen des Lösungsmittels im Vakuum wird der Rückstand an 300 g Kieselgel chromatographiert. Elution mit $CHCl_3/CH_3OH$ von 30 : 1 auf 1 : 1 liefert die entsprechenden Derivate.

Verfahren b und e

725 mg (1 mmol) Nigericin oder Nigericinderivat nach Formel I wird in 30 ml Tetrahydrofuran und 30 ml Wasser suspendiert und mit 4 g $NaIO_4$ für 30 h bei Zimmertemperatur gerührt. Nach dem Abziehen des Tetrahydrofurans im Vakuum wird die wäßrige Phase mit 3 x 50 ml Ethylacetat extrahiert. Nach dem Trocknen über Natriumsulfat wird bis zu einem Sirup eingeengt. Chromatographie an 300 g Kieselgel mit einem Gradienten Chloroform/Methanol 50:1 auf 9:1 (3 Liter) lieferte die entsprechenden Derivate.

Verfahren c

1 mmol Nigericin oder Nigericinderivat wurde in 50 ml Tetrahydrofuran mit 3 mmol Lithiumaluminiumhydrid umgesetzt. Nach vorsichtiger Zugabe von 50 ml Wasser und 10 ml 5 N HCl wurde das THF im Vakuum abdestilliert. Extraktion mit 3 x 50 ml Ethylacetat, Waschen der organischen Phase mit 10 ml 2N HCl, Trocknen über Natriumsulfat und Abdestillieren des Lösungsmittels ergab einen Rückstand, der an 300 g Kieselgel chromatographiert wurde. Elution mit einem Gradienten Chloroform/Methanol von 30:1 auf 9:1

ergab die entsprechenden Alkohole.

Verfahren d

1 mmol Lacton wird in 40 ml Tetrahydrofuran mit 5 mmol eines primären bzw. sekundären Amins 20 h bei Zimmertemperatur gerührt. Nach dem Abdestillieren des Lösungsmittels im Vakuum wird an 300 g Kieselgel chromatographiert. Elution mit Chloroform/Methanol Gradient von 50:1 auf 9:1 ergibt die Amide.

## Antivirale Wirksamkeit

Antivirale Aktivität in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgte die Zugabe von 100 µl einer Suspension von HeLa- bzw. Vero-Zellen in Medium mit 5 % fötalem Kälberserum in einer Zelldichte von 2 x $10^5$ Zellen/ml. Die Ansätze wurden mit 50 µl einer Suspension des jeweiligen Test Virus infiziert, die so eingestellt war, daß die Zellen innerhalb von 72 h einen cytopathogenenj Effekt (Cpe9 zeigten. Die Auswertung erfolgte durch mikroskopische Begutachtung des Zellrasens und photometrische Messung der Neutralrotaufnahme (Farbtest nach Finther). Als MHK wurde die Konzentration des Präparats angenommen (µg/ml), bei der etwa 50 % der Zellen die Infektion überlebten (MIC, minimale Hemmkonzentration).

in Tabelle 3 ist die Wirkung (Angabe des MHK in µg/ml) von verschiedenen erfindungsgemäßen Verbindungen gegen folgende Viren aufgeführt:
Adeno 5, Vaccina, Herpes I, Herpes II, Influenza A, Paramyxo. III, Rhinovirus II

## Antibakterielle Wirksamkeit

Die antibakterielle Wirksamkeit der erfindungsgemäßen Verbindungen wurde im Agar-Verdünnungstest nach Lorian (Antibiotics in Laboratory Medicine, Williams & Wilkins, Baltimore/London, 1980) bestimmt. Dabei wurden die Präparate in einer geometrischen Verdünnungsreihe mit dem Faktor 2 in Müller Hinton Agar verdünnt. Eine Petrischale enthielt nur Müller Hinton Agar und diente zur Kontrolle des Bakterienwachstums. Die Petrischalen wurden dann mit einem Denley-Multipoint-Inokulator, welcher 0,6 µl einer 1 : 100 verdünnten 18-Stunden-Kultur der Testbakterien übertrug, mit den entsprechenden Testbakterien beimpft. Nach 16 bis 18 Stunden Inkubation bei 37°C wurden die Petrischalen makroskopisch auf Bakterienwachstum untersucht.

Die niedrigste Konzentration der erfindungsgemäßen Verbindungen, welche das Bakterienwachstum noch vollständig verhinderte, wurde als MIC (Minimum Inhibitory Concentration) angenommen.

In Tabelle 4 ist die Wirkung (Angabe des MIC in µg/ml) von verschiedenen erfindungsgemäßen Verbindungen gegen folgende Bakterien aufgeführt:
Staph. aureus (SG 511), Staph. aureus (285),
Staph. aureus (503), Strept. pyogenes (308 A),
Strept. pyogenes (77 A), Strept. faecium (D).

Nigericin (Verbindung A) diente als Vergleichssubstanz.

Tabelle 1

| Bsp. | $R_3$ | $R_2$ | $R_1$ | Molgew. | Ausgangs. Verb. | Zeit [h] | Reagens | Verfahren | Temperatur | Ausbeute |
|---|---|---|---|---|---|---|---|---|---|---|
| 1 | | Nigericin | | - | - | - | | - | | - |
| 2 | OH | $CH_2OH$ | H | 726.9 | 1 | 24 | $LiAlH_4$ | c | 25° | 78 % |
| 3 | OH | $CH_2OH$ | $CH_3$ | 763 | 1 | 30 | $CH_3MgCl$ | a | 0° | 65 % |
| 4 | OH | $CH_2OH$ | $C_6H_5$ | 771.0 | | 30 | $C_6H_5MgCl$ | a | 0° | 55 % |
| 5 = III | | | | 692.9 | 1 | 30 | $NaIO_4$ | b | 25° | 82 % |
| 6 | OH | H | H | 696.9 | 5 | 4 | $LiAlH_4$ | c | 25° | 90 % |
| 7 | OH | $CH_3$ | $CH_3$ | 725.0 | 5 | 30 | $CH_3MgCl$ | a | 0° | 82 % |
| 8 | OH | $C_6H_{13}$ | $C_6H_{13}$ | 865.3 | 5 | 30 | $C_6H_{13}MgCl$ | a | 0° | 71 % |
| 9 | | 0 | $NHC_6H_{13}$ | 803 | 5 | 24 | $NH_2C_6H_{13}$ | d | 25° | 68 % |
| 10 | | 0 | H | 694.9 | 2 | 30 | $NaIO_4$ | e | 25° | 87 % |
| 11 | | 0 | $CH_3$ | | 3 | 30 | $NaIO_4$ | e | 25° | 92 % |
| 12 | | 0 | $C_6H_5$ | 803 | 4 | 24 | $NaIO_4$ | e | 25° | 83 % |
| 13 | | 0 | $NHCH_2C_6H_5$ | 800 | 5 | 24 | $NH_2CH_2C_6H_5$ | d | 25° | 72 % |
| 14 | | 0 | $C_2H_5$-N-$C_6H_5$ | 814 | 5 | 24 | $C_2H_5NHC_6H_5$ | d | 25° | 74 % |

* bekannte Verbindungen; u = Grisiorixin

Tabelle 2

| Nr. | CH ber. | CH gef. | FAB MS | $^{13}C$-NMR ppm |
|---|---|---|---|---|
| 1 | - | - | - | - |
| 2 | | | 749 | 176.0, 107.3 |
| 3 | | | 741 | 176.1, 107.6 |
| 4 | C 70.1 H 9.15 | C 69.8 H 9.19 | 793 | |
| 5 | C 67.6 H 9.31 | C 67.8 H 9.11 | 775 | 180.7, 179.9, 109.8 |
| 6 | | | 719 | 175.7, 107.8 |
| 7 | C 67.9 H 10.0 | C 67.6 H 9.8 | 747 | 175.6, 107.7 |
| 8 | C 70.8 H 10.7 | C 70.0 H 10.6 | 887 | 175.5, 107.6 |
| 9 | C 68.8 H 9.3 | C 68.6 H 8.9 | 825 | |
| 10 | | | 715 | 178.4, 108.3. 101.0 |
| 11 | | | | |
| 12 | | | 825 | |
| 13 | C 69.06 H 9.2 | C 68.5 H 9.2 | 823 | 176.4, 175.8, 107.8 |
| 14 | | | 836 | 176.3, 174.1, 107.8 |

Tabelle 3

| MIC in Mμ/ml | | | | | | | |
|---|---|---|---|---|---|---|---|
| Nr. | Adeno 5 | Vaccina | Herpes I | Herpes II | Influenza A | Paramyxo. III | Rhino. II |
| 1 | | | | | | | |
| 2 | < 0.18 | - | 0,49 | < 0.02 | 0,49 | - | 4.44 |
| 3 | < 0.02 | 4.44 | < 0.02 | < 0.02 | 0.16 | - | 0.05 |
| 4 | | | | | | | |
| 5 | 4.44 | 4.44 | 4.44 | 13.3 | 4.44 | - | 40.0 |
| 6 | 1.48 | - | 1.48 | 0.05 | 1.48 | - | 1.48 |
| 7 | < 0.02 | - | < 0.02 | 0.05 | 0.49 | - | 0.05 |
| 8 | 0.16 | 0.05 | 0.16 | 1.48 | - | - | - |
| 9 | 0.05 | 0.16 | 0.16 | - | 0.16 | - | - |
| 10 | < 0.02 | 1.48 | < 0.02 | < 0.02 | 0.49 | - | 0.49 |
| 11 | 0.18 | 0.18 | 0.18 | 0.18 | 0.18 | - | 0.18 |
| 12 | 0.16 | 1.48 | 0.49 | 1.48 | 1.48 | - | - |
| 13 | 0.16 | < 0.02 | 0.49 | 1.48 | 9.44 | - | - |
| 14 | < 0.02 | < 0.02 | < 0.02 | 1.48 | 0.49 | - | 4.44 |
| MHK- minimale Hemmkonzentration | | | | | | | |

Tabelle 4

| Minimale Hemmkonzentration in µg/ml | | | | | | |
|---|---|---|---|---|---|---|
| Nr. | Staph. aureus (S 6511) | Staph. aureus (285) | Staph. aureus (503) | Strept. pyogenes (308A) | Strept. pyogenes (77A) | Strept. faecium (D) |
| 1 | | | | | | |
| 2 | 3.13 | 3.13 | 1.56 | 0.39 | 0.39 | 3.13 |
| 3 | 1.56 | 1.56 | 0.78 | 0.19 | 0.39 | 3.13 |
| 4 | | | | | | |
| 5 | 50.0 | 50.0 | 50.0 | 25.0 | 25.0 | - |
| 6 | 1.56 | 1.56 | 0.78 | 0.195 | 0.39 | 1.56 |
| 7 | 1.56 | 1.56 | 0.78 | 0.195 | 0.39 | 3.13 |
| 8 | 3.17 | 6.25 | 3.13 | 0.39 | 0.39 | 12.5 |
| 9 | 0.78 | 1.56 | 0.78 | 0.098 | 0.098 | 1.50 |
| 10 | 0.098 | 0.098 | 0.098 | 0.049 | 0.049 | 0.319 |
| 11 | 0.025 | 0.098 | 0.013 | 0.013 | 0.013 | 0.391 |
| 12 | 1.56 | 1.56 | 0.78 | 0.098 | 0.098 | 1.56 |
| 13 | 1.56 | 3.13 | 1.56 | 0.39 | 0.39 | 3.13 |
| 14 | 3.13 | 3.13 | 3.13 | 0.78 | 0.78 | 6.25 |

## Ansprüche

1. Verbindungen der Formel I

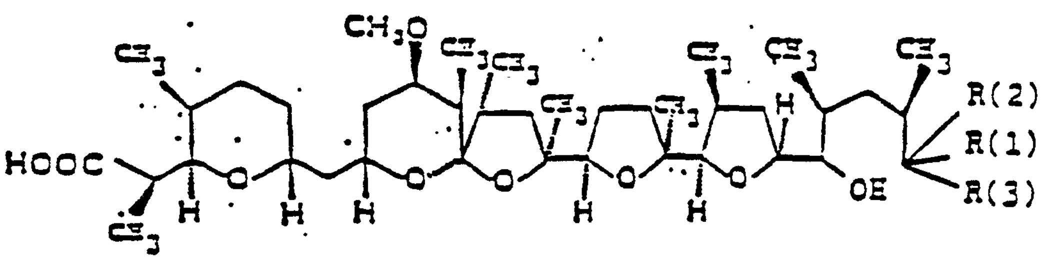

I

in denen
R(1) Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert durch ($C_1$-$C_{10}$)-Alkyl, ($C_1$-$C_{10}$)-Alkoxy oder Phenoxy,
R(2) Wasserstoff, ($C_1$-$C_{18}$)-Alkyl (geradkettig oder verzweigt, gesättigt oder ungesättigt), ($C_3$-$C_8$)-Cycloalkyl, Phenyl, $CH_2OH$, wobei das Phenyl unsubstituiert ist oder substituiert durch ($C_1$-$C_{10}$)-Alkyl, ($C_1$-$C_{10}$)-Alkoxy oder Phenoxy,
R(3) OH,
bedeuten,
oder in der
R(2) und R(3) gemeinsam Sauerstoff und
R(1) Wasserstoff, ($C_1$-$C_{18}$)-Alkyl (geradkettig oder verzweigt, gesättigt oder ungesättigt), ($C_3$-$C_8$)Cycloalkyl, Phenyl (unsubstituiert oder substituiert durch ($C_1$-$C_{10}$)-Alkyl, ($C_1$-$C_{10}$)-Alkoxy oder Phenoxy),

NR(4)R(5) bedeuten, worin
R(4) und R(5) gleich oder verschieden sind und folgende Bedeutung haben:
a) Wasserstoff,
b) ($C_1$-$C_{18}$) Alkyl (gesättigt oder ungesättigt, geradkettig oder verzweigt), wobei die Alkylgruppen unsubstituiert sind oder substituiert mit F, Cl, Br, Phenyl, Naphthyl, Thienyl, oder $CON(R(6))_2$, COOR(6) mit R(6) gleich Wasserstoff, ($C_1$-$C_6$)-Alkyl
c) ($C_3$-$C_8$)-Cycloalkyl,
d) Arylgruppen mit insgesamt 6-20 C-Atomen, worin Aryl Phenyl, Naphthyl, Thienyl bedeutet, welche Systeme entweder unsubstituiert sind oder substituiert mit geradkettigem oder verzweigtem Alkyl mit bis zu 14 C-Atomen, oder F,Cl,Br,I, Nitro, Cyano, Alkoxy, Phenoxy,
sowie die physiologisch verträglichen Salze dieser Verbindungen, mit Ausnahme der Verbindung, in welcher gleichzeitig R(1) Wasserstoff und R(2) $CH_2OH$ und R(3) OH bedeuten; und mit Ausnahme der Verbindungen, in denen gleichzeitig R(2) und R(3) gemeinsam Sauerstoff und R(1) Wasserstoff oder Methyl bedeuten.

2. Verbindungen nach Anspruch 1, dadurch gekennzeichnet, daß mindestens einer der Substituenten folgende Bedeutung hat:
R(1) Wasserstoff, ($C_1$-$C_4$)-Alkyl, Phenyl
R(2) $CH_2OH$
R(3) OH.

3. Verbindungen nach Anspruch I, in der
R(1) und R(2) gleich sind und Wasserstoff, ($C_1$ $C_6$)-Alkyl oder Phenyl,
und
R(3) OH
bedeuten.

4. Verbindung nach Anspruch 1, dadurch gekennzeichnet, daß R(2) und R(3) gemeinsam Sauerstoff und
R(1) NR(4)R(5)
bedeuten,
wobei R(4) und R(5) ($C_1$-$C_6$) Alkyl, Phenyl oder Wassserstoff bedeuten.

5. Verbindung nach Anspruch 1, dadurch gekennzeichnet, daß R(2) und R(3) gemeinsam Sauerstoff und R(1) Wasserstoff, ($C_1$-$C_7$)-Alkyl oder Phenyl bedeuten.

6) Verfahren zur Herstellung einer Verbindung I nach Anspruch 1, dadurch gekennzeichnet, daß man

a)

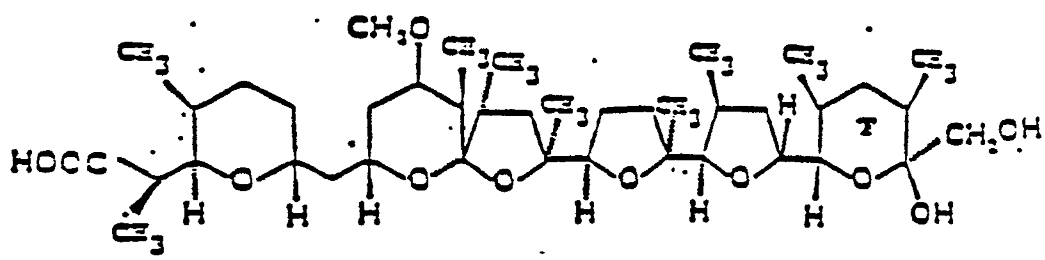

mit einer Verbindung II
R(1)Mg Hal II
umsetzt, in welcher R(1) Wasserstoff, ($C_1$-$C_{18}$)-Alkyl (geradkettig oder verzweigt, gesättigt oder ungesättigt), ($C_3$-$C_8$)-Cycloalkyl, Phenyl, wobei das Phenyl unsubstituiert ist oder substituiert durch ($C_1$-$C_{10}$)-Alkyl, ($C_1$-$C_{10}$)-Alkoxy oder Phenoxy,
und
Hal Cl, Br oder I bedeuten;
oder daß man

b)

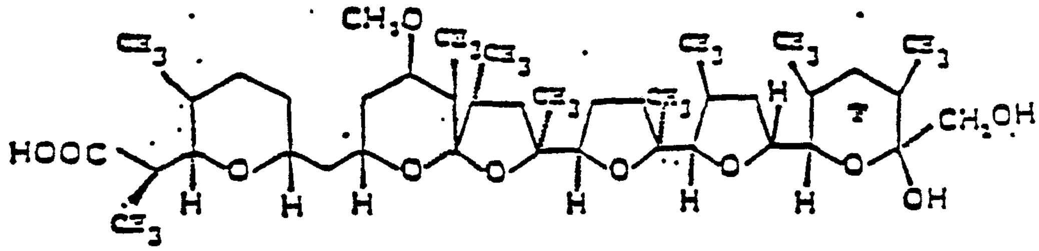

einer Spaltung mit $NaIO_4$ zu Verbindungen

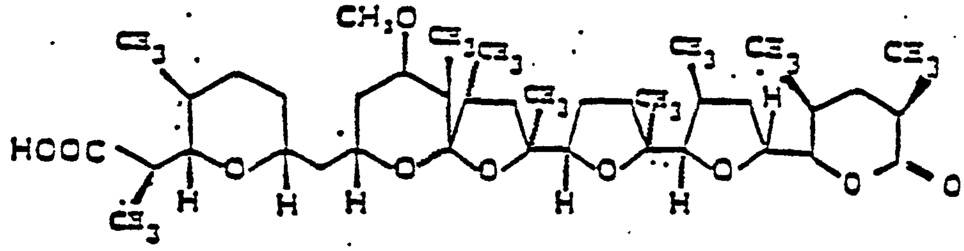

III

unterwirft und diese Verbindungen III analog zu Variante a) mit R(1)Mg Hal (II) zu Verbindungen I umsetzt; oder daß man

c) nach Variante b) Verbindungen III herstellt und diese mit Hydridüberträgern zu Verbindungen I reduziert

oder daß man

d) nach Variante b) Verbindungen III herstellt und diese mit Aminen

HNR(4)R(5)

in denen R(4) und R(5) wie oben definiert sind, zu Amiden der Formel I umsetzt, oder daß man

e) Verbindungen I in denen R(1) gleich ($C_1$-$C_{18}$)-Alkyl (geradkettig oder verzweigt, gesättigt oder ungesättigt), ($C_3$-$C_8$)-Cycloalkyl oder Phenyl ist, wobei das Phenyl unsubstituiert ist oder substituiert durch ($C_1$-$C_{10}$)-Alkyl, ($C_1$-$C_{10}$)-Alkoxy oder Phenoxy, bedeutet,

$R_2 = CH_2OH$ ist und R(1) gleich OH ist,

einer Spaltung mit Natriumperiodat unterwirft, wobei Verbindungen I mit R(2) und R(3) gemeinsam gleich Sauerstoff und R(1) wie oben definiert entstehen und daß man gegebenenfalls anschließend die Verbindungen der Formeln I oder III in ihre physiologisch verträglichen Salze überführt.

7. Verbindung III

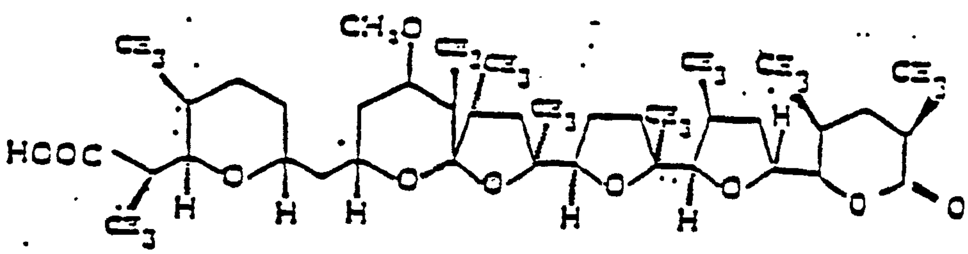

III

8. Verbindung I nach Anspruch 1 sowie deren physiologisch verträgliche Salze zur Anwendung als Arzneimittel

9. Verbindung I nach Anspruch 1 sowie deren physiologisch verträgliche Salze zur Anwendung als antibakteriell und antiviral wirkendes Arzneimittel

10. Verbindung III nach Anspruch 6 sowie deren physiologisch verträgliche Salze zur Anwendung als Arzneimittel

11. Verbindung III nach Anspruch 6 sowie deren physiologisch verträgliche Salze zur Anwendung als antibakteriell und antiviral wirkendes Arzneimittel

12. Verbindung I mit R(1) gleich H, R(2) gleich $CH_2OH$ und R(3) gleich OH sowie deren physiologisch verträglichen Salze zur Anwendung als antiviral wirkendes Arzneimittel.

13. Verbindung I mit R(2) und R(3) gemeinsam gleich Sauerstoff und R(1) gleich Wasserstoff oder Methyl sowie deren physiologisch verträglichen Salze zur Anwendung als antiviral wirkendes Arzneimittel.

Europäisches Patentamt

# EUROPÄISCHER RECHERCHENBERICHT

Nummer der Anmeldung

EP 89 10 5353

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 907 976 (TEISHO PHARMACEUTICAL CO. LTD.)<br>* Ansprüche; Seiten 3,4; Seite 6, Absatz 2; Seite 7, Zeilen 11-18 * | 1,2,6,8,9 | C 07 D 493/10<br>A 61 K 31/35 //<br>(C 07 D 493/10<br>C 07 D 311:00<br>C 07 D 307:00 ) |
| P,A | FR-A-2 605 221 (SANOFI)<br>* Ansprüche 1,2; Beispiel 1 * | 6,7,10,11 | |
| A | JOURNAL OF THE CHEMICAL SOCIETY, PERKIN TRANSACTIONS II<br>Nr. 9, 1975, Seiten 907-911; P. GACHON et al.: "Grisorixin, an Ionophorous Antibiotic of the Nigericin Group. Part IV. Complexation of Monovalent Cations"<br>* Seite 907, Verbindung (2) * | 1,8 | |
| D,A | US-A-3 832 358 (J. W. CHAMBERLIN)<br>* Anspruch 1; Zusammenfassung * | 1,8 | |
| P,X | CHEMICAL ABSTRACTS<br>Band 109, Nr. 23, 5. Dezember 1988, Seite 375, Zusammenfassung Nr. 208017n, Columbus, Ohio, USA; A. M. DELORT et al.: "Microbial conversion of nigericin in three successive steps, by Sebekia benihana"; & J. Antibiot. 1988, 41(7), 916-924 | 1,2,6 | **RECHERCHIERTE SACHGEBIETE (Int. Cl.5)**<br>C 07 D 493/00<br>C 07 H 19/00 |

Der vorliegende Recherchenbericht wurde für alle Patentansprüche erstellt

| Recherchenort | Abschlußdatum der Recherche | Prüfer |
|---|---|---|
| BERLIN | 26-06-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)